# EUROPEAN PATENT APPLICATION

(11) **EP 4 813 385 A1**
(43) Date of publication of application: **30.09.2026**
(21) Application number: 24870995.8
(22) Date of filing: 27.09.2024
(51) Int. Cl.: A61K 31/439, A61K 45/06, A61K 9/08, A61P 27/02, A61P 27/10

(54) **OPHTHALMIC PHARMACEUTICAL COMPOSITION AND USE THEREOF**

(30) Priority: 28.09.2023 CN 202311275699
(71) Applicant: Suzhou Pro-Heal Pharmaceuticals Technology Co., Ltd., Suzhou, Jiangsu 210000 (CN)
(72) Inventor: PANG, Liang, Suzhou, Jiangsu 210000 (CN); WANG, Qifang, Suzhou, Jiangsu 210000 (CN); MA, Lin, Suzhou, Jiangsu 210000 (CN); GUO, Shuhua, Suzhou, Jiangsu 210000 (CN); ZHOU, Wenchao, Suzhou, Jiangsu 210000 (CN)
(74) Representative: Elzaburu S.L.P.
(86) International application number: PCT/CN2024/121849
(87) International publication number: WO 2025/067458

(57) **Abstract**

An ophthalmic pharmaceutical composition and a use thereof. The ophthalmic pharmaceutical composition comprises one or two of a choline receptor agonist, an anticholinesterase drug, an α-adrenergic receptor agonist or a choline receptor antagonist. The ophthalmic pharmaceutical composition has a very good curative effect on myopia, is quick to take effect, has high patient compliance, has no specific use time and occasion, and changes the conventional mode of using myopia drugs at night.

## Description

### TECHNICAL FIELD

The present disclosure relates to the technical field of medicines and particularly relates to an ophthalmic pharmaceutical composition and use thereof.

### BACKGROUND

Myopia is a symptom in which the eyes cannot see objects far away but can see objects near. Under the premise of refraction quiescence, distant objects cannot converge at the retina, but form a focus in front of the retina, thereby causing vision distortion and leading to blurring of the distant objects. The myopia is divided into two categories, i.e., refractive and axial myopia. Among them, the refractive myopia is the most severe. The refractive myopia can reach over 600 degrees, namely high myopia.

Myopia refers to a refractive condition in which parallel light rays, upon refraction through the ocular dioptric system, converge to a focal point anterior to the retina when the eyes are not adjusted. Therefore, the myopic eye cannot clearly see a distant target. If the target is gradually moved towards the eye, the emitted rays scatter to a certain degree to the eye, and the formed focus moves backwards. When the target moves to a certain point in front of the eye, the target can be clearly seen only. As the object point moves closer to the eye, the myopic eye has a higher degree.

The myopia is caused by a disproportionate relationship between the axial length of the eye and the focal length. The focal length is mainly determined by the curvature of the cornea and crystalline lens. An increase in the axis oculi length or the curvature of the cornea and crystalline lens occurs in the progression of the myopia. When the axis oculi is too long, the shape of the crystalline lens cannot be proportionally changed to create a depth of field that focuses on the retina, primarily because the function of the ciliary muscle limits the variation in the thickness of the crystalline lens.

Atropine, a non-selective cholinoceptor antagonist, has been clinically proven for its inhibitory effect on the myopia. However, its mechanism of action is not yet fully understood, and it is currently believed to act through a variety of mechanisms, such as reduction of axis oculi length, remodeling of scleral tissue, and inhibition of expression of γ-aminobutyric acid transporter in the retina. However, atropine can only prevent and control the progression of myopia in adolescents, but has no effect on adult patients with myopia. Besides, an atropine eye drop has side effects and causes blurred vision. Therefore, the atropine can only be dropped before sleep, thereby also causing inconvenience. Furthermore, an atropine drug further associated with adverse reactions including photophobia and myopia blurring. By inducing paralysis of accommodation, thereby causing difficulty in reading books and newspapers of children patients and also causing visual problems such as photophobia and myopia blurring.

In view of the vacancy of the existing drugs for clinical treatment of adults with myopia and the side effects of the atropine for treating adolescents with myopia, it is urgently needed to develop an ophthalmic preparation which can be used for a long time, featuring stability, high patient compliance, and small side effect, and is convenient to use, and is used for treating and ameliorating distance vision of both adults and adolescents.

### SUMMARY

Aiming at the defects of the drugs for myopia, the present disclosure uses cevimeline in preparing an ophthalmic preparation for treating and ameliorating myopia, and provides the ophthalmic preparation used for treating and ameliorating distance vision of adults and adolescents. The ophthalmic preparation ameliorates myopia, can be used for a long time, has good stability, high patient compliance, and small side effect, and is convenient to use.

In order to achieve the aforementioned objectives of the present disclosure, a specific technical solution used by the present disclosure is as follows:

In the first aspect, the present disclosure provides an ophthalmic preparation for treating and/or ameliorating myopia, wherein the ophthalmic preparation comprises cevimeline and the concentration of the cevimeline is 0.1-10 wt%.

In an optional embodiment, the ophthalmic preparation further comprises at least one of another cholinoceptor agonist other than cevimeline, an anticholinesterase drug, an α-adrenoceptor agonist, or a cholinoceptor antagonist.

Wherein the cholinoceptor agonist comprises acetylcholine, methacholine, carbachol, bethanechol chloride, pilocarpine, aceclidine, cevimeline, xanomeline, sabcomeline, methacholine bromide, milameline or arecoline, or a derived compound having the same function thereof.

Wherein the anticholinesterase drug comprises neostigmine, mestinon, physostigmine, edrophonium chloride, ambenonium chloride, demecarium bromide, galantamine, tacrine, huperzine A or a derived compound possessing the same function thereof, and is optionally neostigmine whose concentration is 0.0001-0.5 wt%.

Wherein the α-adrenoceptor agonist comprises norepinephrine, metaraminol, phenylephrine, methoxamine, oxymetazoline, naphazoline, xylometazoline, apraclonidine, dexmedetomidine, brimonidine, dopamine, ephedrine, pseudoephedrine, mephentermine, guanfacine, Iopidine, tetrahydrozoline, xylazine, tizanidine, moxonidine, rilmenidine or derivatives thereof possessing the same function thereof, and is optionally brimonidine whose concentration is 0.05-1.0 wt%.

Wherein the cholinoceptor antagonist comprises atropine, atropine methyl bromide, ipratropium bromide, anisodamine, pirenzepine, mecamylamine, pancuronium bromide, propantheline bromide, benactyzine or a derived compound possessing the same function thereof, and is optionally atropine whose concentration is 0.01-1.0 wt%.

In an optional embodiment, the ophthalmic preparation is used for ameliorating, alleviating, inhibiting and/or relieving pupil photophobia, myopia blurring and/or accommodative eye fatigue in both adolescents and adult subjects.

In another optional embodiment, the ophthalmic preparation is further used for ameliorating and improving distance visual acuity in both adolescents and adult subjects.

In an optional embodiment, the ophthalmic preparation further comprises a pharmaceutically acceptable carrier and/or excipient, which includes but is not limited to one or more of a pH regulator, an osmotic pressure regulator or a buffer.

In an optional embodiment, the dosage form of the ophthalmic preparation includes an eye drop, an eye gel, an eye ointment, an eye patch, an eye film, an eye wash or an intraocular injection.

In the second aspect, the present disclosure provides use of cevimeline or a derivative thereof in the manufacture of a medicament for treating or preventing myopia. The use comprises: (a) ameliorating, alleviating, inhibiting and/or relieving pupil photophobia, myopia blurring and/or accommodative eye fatigue in adolescents and adults, and/or (b) ameliorating and improving distance vision in both adolescents and adults.

In the third aspect, the present disclosure provides use of the ophthalmic preparation of the first aspect in the manufacture of a medicament possessing the above functions.

In the fourth aspect, the present disclosure provides a method for preparing the ophthalmic preparation according to the first aspect. The preparation method comprises adding the cevimeline or the derivative thereof into a solvent and making up to a volume to obtain the ophthalmic preparation.

In the fifth aspect, the present disclosure provides a method for treating or preventing myopia, comprising: administering to a subject a therapeutically effective amount of the ophthalmic preparation or medicament according to the present disclosure.

Compared with the prior art, the present disclosure provides the following beneficial effects:

The present disclosure uses cevimeline in the manufacture of a medicament for treating and preventing myopia. Single use or combined use with other pharmaceutical components associated with the treatment of eye diseases achieves effective amelioration in subjects with myopia. Besides, the technical effect of quickly and durably adjusting the distance vision can be realized by adjusting different pharmaceutical components and different concentrations.

The present disclosure further confirms that the combination of cevimeline and atropine significantly mitigates the adverse effects of the atropine and can improve the upper limit of the safe dose of the atropine, thereby further improving the treatment or prevention effect of myopia by using the atropine with higher concentration. The elimination of the side effects improves patient compliance and enables the patients to use safely for a long time.

Furthermore**,** it is verified that the ophthalmic preparation provided by the present disclosure breaks the limitation that the atropine is effective only for adolescents, and also achieves a significant amelioration effect for adults. Besides, through tests of improvement of diopter and axis oculi, the present disclosure confirms that the combined use of the cevimeline and the atropine has a synergistic effect on reducing the diopter and the axis oculi.

### DETAILED DESCRIPTION OF THE EMBODIMENTS

The technical solutions of examples of the present disclosure are further described in detail, and the described examples are only a part of the present disclosure for explaining the present disclosure, but not for limiting the present disclosure. Therefore, other examples obtained by those skilled in the art without creative efforts all belong to the protection scope of the present disclosure.

### (I) Terms and definitions

Unless otherwise specified in the present disclosure, the term "cholinoceptor agonist" refers to a drug that can directly bind to and agonize an acetylcholine (ACh) M receptor (muscarinic receptor) and/or an acetylcholine (ACh) N receptor (nicotinic receptor), including a direct-acting cholinomimetic drug which belongs to a direct-acting parasympathomimetic. According to different binding receptors, the cholinoceptor agonist can be divided into three specific types:
(1) Choline esters: drug components capable of simultaneously acting on M receptors and N receptors, including acetylcholine and a plurality of pharmaceutical components with high consistency with the acetylcholine in structure and function, such as methacholine and carbachol.
(2) Alkaloids: drug components selectively acting on M receptors, including synthetic substances with an agonistic effect on acetylcholine receptor, such as pilocarpine and arecoline.
(3) Nicotine: a drug component selectively agonizing N receptors. However, the effect of the nicotine is relatively complex and bidirectional: the nicotine initially plays an agonistic effect on the N receptors, but plays an inhibitory effect on the N receptors as time goes on and maintains the effect continuously.

Unless otherwise specified in the present disclosure, the term "anticholinesterase drug" refers to a class of drug molecules capable of binding to cholinesterase (AChE) and inhibiting the activity of the cholinesterase, resulting in accumulation of acetylcholine and production of a cholinomimetic effect. According to the binding degree of the anticholinesterase drug and the cholinesterase, the anticholinesterase drug can be divided into an easily reversible anticholinesterase drug and a difficultly reversible anticholinesterase drug.

Unless otherwise specified in the present disclosure, the term "α-adrenoceptor agonist" refers to a class of drug molecules capable of activating an α1 adrenoceptor and an α2 adrenoceptor. Generally, the class of drug molecules is less agonistic at a β1 receptor and has little effect at a β2 receptor.

Unless otherwise specified in the present disclosure, the term "cholinoceptor antagonist", also known as a cholinoceptor blocking drug, refers to a class of drug molecules capable of binding to a cholinoceptor and interfering with the binding of Ach or a cholinoceptor agonist to the cholinoceptor, thereby antagonizing the cholinomimetic effect. According to different blocking receptors, the cholinoceptor antagonist can be classified as an M receptor antagonist such as atropine and an analogous alkaloid thereof (anisodamine, etc.), or an atropine substitute (propantheline bromide, benactyzine, etc.) which binds to a cholinoceptor and competitively blocks the binding of ACh to the receptor, such that the ACh cannot cause the depolarization of ganglion cells, thereby blocking the transmission of nerve impulses in the ganglions.

Unless otherwise specified in the present disclosure, the term "effective amount" or "effective dose" refers to an amount of a drug capable of producing a therapeutic effect on humans and/or animals and acceptable to humans and/or animals. For example, a therapeutically or pharmaceutically effective amount of a drug refers to an amount of a drug required to produce a desired therapeutic effect which can be reflected by results of clinical trials, model animal studies, and/or *in-vitro* studies. The pharmaceutically effective amount depends on several factors including (but not limited to): characteristic factors of a treatment subject (such as the height, weight, sex, age and medication history of the treatment subject), and severity of the disease. In a specific embodiment of the present disclosure, the "effective amount" refers to an amount sufficient to inhibit, slow or prevent the development of myopia in a subject.

Unless otherwise specified in the present disclosure, the term "ameliorating, relieving, or treating" refers to the medical management of a patient for the purpose of curing, ameliorating, stabilizing, or preventing a disease, a pathological state, or a disorder. The term includes an active treatment, that is, a treatment for the purpose of specially ameliorating the disease, the pathological state, or the disorder, and also includes an etiological treatment, that is, a treatment for the purpose of removing the cause of the relevant disease, the pathological state, or the disorder. Moreover, the term further includes a palliative treatment, that is, a treatment designed to alleviate symptoms rather than cure a disease, a pathological state, or a disorder. The term further includes a prophylactic treatment, that is, a treatment for the purpose of minimizing or partially or completely inhibiting the development of the relevant disease, the pathological state, or the disorder; and a supportive treatment, that is, a treatment for supplementing another specific treatment for the purpose of ameliorating the relevant disease, the pathological state, or the disorder. In a specific embodiment of the present disclosure, the "ameliorating, relieving, or treating" refers to relieving the severity of symptoms of an eye condition that adversely affects visual acuity. In particular, the ophthalmic preparation or the pharmaceutical composition described herein can be used to ameliorate or treat symptoms of myopia. A patient can visually focus on an object at a distance by using the preparation.

### (II) Specific embodiments

In some aspects, the present disclosure provides an ophthalmic preparation for treating and/or ameliorating myopia, wherein the ophthalmic preparation includes cevimeline.

In some embodiments of the present disclosure, the ophthalmic preparation further comprises: one or more other cholinoceptor agonists other than cevimeline, or the ophthalmic preparation may further comprise one or more anticholinesterase drugs, or the ophthalmic preparation may further comprise one or more α-adrenoceptor agonists, or the ophthalmic preparation may further comprise one or more cholinoceptor antagonists.

In an optional embodiment, the ophthalmic preparation comprises cevimeline and another cholinoceptor agonist, such as cevimeline and acetylcholine.

In an optional embodiment, the ophthalmic preparation comprises cevimeline and at least two or more additional cholinoceptor agonists.

In an optional embodiment, the ophthalmic preparation includes cevimeline and at least one anticholinesterase drug, such as cevimeline and neostigmine.

In an optional embodiment, the ophthalmic preparation comprises cevimeline and at least two or more additional anticholinesterase drugs.

In an optional embodiment, the ophthalmic preparation comprises cevimeline and at least one α-adrenoceptor agonist, such as cevimeline and brimonidine.

In an optional embodiment, the ophthalmic preparation comprises cevimeline and at least two or more additional α-adrenoceptor agonists.

In an optional embodiment, the ophthalmic preparation comprises cevimeline and other cholinoceptor antagonists, such as cevimeline and atropine.

In an optional embodiment, the ophthalmic preparation comprises cevimeline and at least two or more additional cholinoceptor antagonists.

In an optional embodiment, the ophthalmic preparation further comprises at least two kinds selected from: cholinoceptor agonist, anticholinesterase drug, or an α-adrenoceptor agonist. Such as a combination of the cevimeline, one or more other cholinoceptor agonists, and one or more anticholinesterase drugs; a combination of the cevimeline, one or more other cholinoceptor agonists, and one or more anticholinesterase drugs; a combination of the cevimeline, one or more cholinoceptor antagonists, and one or more anticholinesterase drugs; and other combinations that are not exhaustive.

In some embodiments of the present disclosure, the cholinoceptor agonist comprises acetylcholine, methacholine, carbachol, bethanechol chloride, pilocarpine, aceclidine, cevimeline, xanomeline, sabcomeline, methacholine bromide, milameline or arecoline, or a derived compound possessing the same function thereof.

In some embodiments of the present disclosure, the anticholinesterase drug comprises neostigmine, mestinon, physostigmine, edrophonium chloride, ambenonium chloride, demecarium bromide, galantamine, tacrine, huperzine A or a derived compound possessing the same function thereof.

In some embodiments of the present disclosure, the α-adrenoceptor agonist comprises norepinephrine, metaraminol, phenylephrine, methoxamine, oxymetazoline, naphazoline, xylometazoline, apraclonidine, dexmedetomidine, brimonidine, dopamine, ephedrine, pseudoephedrine, mephentermine, guanfacine, Iopidine, tetrahydrozoline, xylazine, tizanidine, moxonidine, rilmenidine or a derived compound possessing the same function thereof.

In some embodiments of the present disclosure, the cholinoceptor antagonist comprises atropine, atropine methyl bromide, ipratropium bromide, anisodamine, pirenzepine, mecamylamine, pancuronium bromide, propantheline bromide, benactyzine or a derived compound possessing the same function thereof.

In some embodiments of the present disclosure, the derived compound comprises a cross-linked compound, a stereoisomer, an organic solution, or a pharmaceutically acceptable organic salt comprising the cholinoceptor agonist, the anticholinesterase drug, the α-adrenoceptor agonist, or the cholinoceptor antagonist.

The organic salt may be a salt formed by a cholinoceptor agonist, an anticholinesterase drug, an α-adrenoceptor agonist, or a cholinoceptor antagonist with a cation, for example, an ammonium salt, an alkali metal salt (comprising a sodium salt, a lithium salt and a potassium salt), and an alkaline earth metal salt (comprising a calcium salt and a magnesium salt). The organic salt can further be a salt formed by a cholinoceptor agonist, an anticholinesterase drug, an α-adrenoceptor agonist, or a cholinoceptor antagonist with an organic alkali, such as an organic amine, benzathines, dicyclohexylamines, hydrabamines formed with N,N-bis(dehydroabietyl)ethylenediamine, N-methyl-D-glucamine, and tert-butylamine. The organic salt can further be a salt formed with an amino acid such as arginine, lysine, and aspartate. The organic salt can also be a salt formed with an organic acid such as acetate, adipate, alginate, ascorbate, benzoate, benzenesulfonate, bisulfate, borate, butyrate, citrate, camphanic acid, camphorsulfonate, cyclopentanepropionate, hydrochloride, hydrobromide, hydroiodide, 2-hydroxyethanesulfonate, lactate, maleate, methanesulfonate, 2-naphthylmethanesulfonate, nicotinate, nitrate, oxalate, pectate, persulfate, digluconate, dodecylsulfate, ethanesulfonate, fumarate, glucoheptonate, glycerophosphate, hemisulfate, heptanoate, hexanoate, 3-phenylpropionate, phosphate, picrate, pivalate, propionate, salicylate, succinate, sulfate, sulfonate, tartrate, thiocyanate, tosylate, or undecanoate.

In some embodiments of the present disclosure, the ophthalmic preparation comprises cevimeline in a concentration of 0.1-10 wt%, including but not limited to,0.1 wt%, 0.2 wt%, 0.3 wt%, 0.4 wt%, 0.5 wt%, 0.6 wt%, 0.7 wt%, 0.8 wt%, 0.9 wt%, 1.0 wt%, 1.5 wt%, 2.0 wt%, 2.5 wt%, 3.0 wt%, 3.5 wt%, 4.0 wt%, 4.5 wt%, 5.0 wt%, 5.5 wt%, 6.0 wt%, 6.5 wt%, 7.0 wt%, 7.5 wt%, 8.0 wt%, 8.5 wt%, 9.0 wt%, 9.5 wt%, or 10 wt%, preferably 0.5-8 wt%, further preferably 1-5 wt%, further preferably 2-3 wt%, and further preferably 2.25%.

In some embodiments of the present disclosure, the anticholinesterase drug is neostigmine in a concentration of 0.0001-0.5 wt%, including, but not limited to, 0.0001 wt%, 0.0002 wt%, 0.0003 wt%, 0.0004 wt%, 0.0005 wt%, 0.0006 wt%, 0.0007 wt%, 0.0008 wt%, 0.0009 wt%, 0.0010 wt%, 0.0015 wt%, 0.0020 wt%, 0.0025 wt%, 0.0030 wt%, 0.0035 wt%, 0.0040 wt%, 0.0045 wt%, 0.0050 wt%, 0.0055 wt%, 0.0060 wt%, 0.0065 wt%, 0.0070 wt%, 0.0075 wt%, 0.0080 wt%, 0.0085 wt%, 0.0090 wt%, 0.0095 wt%, 0.010 wt%, 0.015 wt%, 0.020 wt%, 0.025 wt%, 0.030 wt%, 0.035 wt%, 0.040 wt%, 0.045 wt%, 0.050 wt%, 0.055 wt%, 0.060 wt%, 0.065 wt%, 0.070 wt%, 0.075 wt%, 0.080 wt%, 0.085 wt%, 0.090 wt%, 0.095 wt%, 0.10 wt%, 0.15 wt%, 0.20 wt%, 0.25 wt%, 0.30 wt%, 0.35 wt%, 0.40 wt%, 0.45 wt%, or 0.50 wt%, preferably 0.0001-0.1 wt%, further preferably 0.0001-0.05 wt%, further preferably 0.0001-0.01 wt%, further preferably 0.0001-0.001 wt%, and further preferably 0.0001 wt%.

In some embodiments of the present disclosure, the α-adrenoceptor agonist is brimonidine in a concentration of 0.05-1.0 wt%, including, but not limited to,0.05 wt%, 0.06 wt%, 0.07 wt%, 0.08 wt%, 0.09 wt%, 0.10 wt%, 0.15 wt%, 0.20 wt%, 0.25 wt%, 0.30 wt%, 0.35 wt%, 0.40 wt%, 0.45 wt%, 0.50 wt%, 0.55 wt%, 0.60 wt%, 0.65 wt%, 0.70 wt%, 0.75 wt%, 0.80 wt%, 0.85 wt%, 0.90 wt%, 0.95 wt%, or 1.0 wt%, preferably 0.05-0.5 wt%, further preferably 0.05-0.2 wt%, further preferably 0.1-0.2 wt%, and further preferably 0.15 wt%.

In some embodiments of the present disclosure, the cholinoceptor antagonist is atropine in a concentration of 0.01-1.0 wt%, including, but not limited to, 0.01 wt%, 0.02 wt%, 0.03 wt%, 0.04 wt%, 0.05 wt%, 0.06 wt%, 0.07 wt%, 0.08 wt%, 0.09 wt%, 0.10 wt%, 0.15 wt%, 0.20 wt%, 0.25 wt%, 0.30 wt%, 0.35 wt%, 0.40 wt%, 0.45 wt%, 0.50 wt%, 0.55 wt%, 0.60 wt%, 0.65 wt%, 0.70 wt%, 0.75 wt%, 0.80 wt%, 0.85 wt%, 0.90 wt%, 0.95 wt%, or 1.0 wt%, preferably 0.01-0.5 wt%, preferably 0.01-0.1 wt%, preferably 0.03-0.07 wt%, and further preferably 0.05 wt%.

In some embodiments of the present disclosure, the ophthalmic preparation provided by the present disclosure is used for ameliorating, alleviating, inhibiting and/or relieving pupil photophobia, blurred near vision and/or accommodative eye fatigue in both adolescents and adults. In some other embodiments of the present disclosure, the ophthalmic preparation provided by the present disclosure can further be used for ameliorating and improving distance vision in both adolescents and adults.

In some embodiments, the ophthalmic preparation provided by the present disclosure is administered to one or both eyes of a subject with the symptoms of myopia to improve patient's ability to focus on an object at a distance, including an object around a normal reading distance. In some embodiments, administration of the ophthalmic preparation provided by the present disclosure can substantially improve patient's distance vision, independent of other treatment methods. For example, the administration of the ophthalmic preparation provided by the present disclosure can enable a patient to focus on an object at a distance around a normal reading distance without the use of corrective lenses or corrective eye surgery. For adolescent and adult subjects suffering from myopia, the administration of the ophthalmic preparation provided by the present disclosure can alleviate symptoms related with myopia, including making distance vision sensitivity possible without the use of corrective lenses or glasses, as well as alleviating photophobia or blurred vision caused by myopic symptoms themselves, or the administration of other drugs.

In some embodiments, the ophthalmic preparation provided by the present disclosure is administered to a subject with myopic symptoms to facilitate visual acuity of near distance, such that the patient can read without relying on corrective treatments such as bifocal/multifocal lenses or monocular contact lenses.

In some embodiments, the ophthalmic preparation provided by the present disclosure is administered to one or both eyes of a patient to provide treatment for myopia as an alternative to a corrective eye surgery. For example, when a patient cannot receive a corrective eye surgery to treat myopia, the ophthalmic preparation provided by the present disclosure can be administered to a patient with myopic symptoms. Alternatively, after a patient has undergone a corrective eye surgery for myopia, the ophthalmic preparation provided by the present disclosure can be administered to a patient who continues to have myopic symptoms.

In some embodiments, after undergoing a corrective eye surgery for distance vision at a younger age, the ophthalmic preparation disclosed herein is administered to one or both eyes of a patient to reduce the decline in sensitivity of distance vision.

In some embodiments, the administration of the ophthalmic preparation disclosed herein is beneficial for ameliorating myopic symptoms in a patient who has undergone a reversal of a previously accepted corrective eye surgery for myopia. For example, after reversal or regression of a previously received single eye laser surgery directed to the treatment of myopia, administration of the ophthalmic preparation to a patient can reconstruct binocular vision distance.

In some embodiments, after a patient has undergone a corrective surgery for treating an ocular condition other than myopia (for example, a corrective eye surgery for treating cataracts), the ophthalmic preparation disclosed herein is administered to a patient to improve patient's ability to focus on a distant object.

In some other embodiments of the present disclosure, the ophthalmic preparation further comprises a pharmaceutically acceptable carrier and/or excipient. The pharmaceutically acceptable carrier and/or excipient is recognized in the art, and comprises, for example, a pharmaceutically acceptable material, composition or excipient, such as a liquid or a solid filler, a pH regulator, an osmotic pressure regulator, a diluent, a solvent, or an encapsulating material, involved in carrying or transporting any subject composition from one organ or a portion of the body to another organ or a portion of the body. Each carrier must be "acceptable" in the sense of being compatible with the other ingredients of a host preparation and is harmless to patients.

In some other embodiments, the ophthalmic preparation of the present disclosure comprises a preservative such as benzalkonium chloride, sorbic acid, an oxychloro complex, citric acid, chlorobutanol, thiomersalate, phenylmercuric acetate, ethylenediaminetetraacetic acid disodium salt, phenylmercuric nitrate, perborate, or benzyl alcohol.

In some embodiments, the ophthalmic preparation of the present disclosure does not comprise any preservatives.

In some embodiments, the pH regulator in the ophthalmic preparation of the present disclosure is selected from one or more of hydrochloric acid, boric acid, phosphoric acid, citric acid, sodium hydroxide, potassium hydroxide, an acetate buffer, a citrate buffer, a phosphate buffer, or a borate buffer.

In some embodiments, the osmotic pressure regulator in the ophthalmic preparation of the present disclosure is selected from one or more of sodium chloride, sodium sulfate, potassium chloride, magnesium chloride, mannitol, mannose, or dextrose.

In some embodiments, the ophthalmic preparation of the present disclosure includes a buffer, and the buffer comprises one or more of carbonates, phosphates, citrates, acetates, borates, barbiturates, and 3-(N-morpholine) propanesulfonates.

In some embodiments of the present disclosure, the dosage form of the ophthalmic preparation provided by the present disclosure comprises an eye drop, an eye gel, an eye ointment, an eye patch, an eye film, an eye wash or an intraocular injection. The dosage form of the ophthalmic preparation is matched with different administration modes and administration doses, and is particularly influenced by factors such as age, body weight, sex, morbid state, diet, excretion rate, response sensitivity and the like of a subject or a patient, various prescriptions can be administered, and a skilled doctor can generally easily determine the dosage form of a prescription, and the administration dose and administration mode of the prescription effective for desired treatment or prevention. In addition, those skilled in the art clearly know that the administration mode, the administration dosage form, and the administration dose are influenced by various factors such as age, body weight, sex, morbid state, diet, administration time, excretion rate, response sensitivity and the like of a patient. Therefore, the administration mode, the administration dosage form, and the administration dose of the ophthalmic preparation or the pharmaceutical composition in the present disclosure are not limited to those described in the examples of the present disclosure.

Further, the subject refers to any animal, and further refers to human and non-human animals. The non-human animals include all vertebrates, e.g., mammals, such as non-human primates (particularly higher primates), sheep, dogs, rodents (e.g., mice or rats), guinea pigs, goats, pigs, cats, rabbits, cattle, and any domestic animals or pets; and non-mammals, such as chickens, amphibians, reptiles, and the like. In a specific embodiment of the present disclosure, the subject is preferably a human.

In another aspect, the present disclosure provides use of the above-mentioned cevimeline or the derivative thereof in the manufacture of a medicament for treating or preventing myopia. The use comprises: (a) ameliorating, alleviating, inhibiting and/or relieving pupil photophobia, blurred near vision and/or accommodative eye fatigue in adolescents and adults, and/or (b) ameliorating and improving distance vision in adolescents and adults.

In some embodiments, the cevimeline or the derivative thereof is used alone in the manufacture of a medicament for treating or preventing myopia.

In some other embodiments, the cevimeline or the derivative thereof is used in combination with at least one of another cholinoceptor agonist other than cevimeline, an anticholinesterase drug, an α-adrenoceptor agonist, or a cholinoceptor antagonist in the manufacture of a medicament for treating or preventing myopia.

In some embodiments, a medicament for treating or preventing myopia using the cevimeline as a main effective ingredient further comprises a pharmaceutically acceptable carrier and/or excipient such as at least one of a pH regulator, an osmotic pressure regulator or a buffer.

In another aspect, the present disclosure provides use of the ophthalmic preparation in the manufacture of a medicament for treating or preventing myopia. The use comprises: (a) ameliorating, alleviating, inhibiting and/or relieving pupil photophobia, blurred near vision and/or accommodative eye fatigue in adolescents and adults, and/or (b) ameliorating and improving distance vision in adolescents and adults.

In another aspect, the present disclosure provides a method for preparing the ophthalmic preparation of the present disclosure. The method comprises adding the cevimeline or the derivative thereof into a solvent and making up to a volume to obtain the ophthalmic preparation. When the cevimeline or the derivative thereof is used in combination with other effective components such as at least one of another cholinoceptor agonist, an anticholinesterase drug, an α-adrenoceptor agonist, or a cholinoceptor antagonist, other effective components may be incorporated prior to diluting the mixture to a final volume.

Preferably, prior to diluting the mixture to a final volume, a pharmaceutically acceptable carrier and/or excipient such as at least one of a pH regulator, an osmotic pressure regulator or a buffer is further required to be added.

In another aspect, the present disclosure further provides a method for treating or preventing myopia, comprising administering to a subject a therapeutically effective amount of the ophthalmic preparation or drug of the present disclosure. The dosage form of the ophthalmic preparation is matched with different administration modes and administration doses, and is particularly influenced by factors such as age, body weight, sex, morbid state, diet, excretion rate, response sensitivity and the like of a subject or a patient, various prescriptions can be administered, and a skilled doctor can generally easily determine the dosage form of a prescription, and the administration dose and administration mode of the prescription effective for desired treatment or prevention.

In another aspect, the present disclosure further provides a medicament for treating or preventing myopia, comprising cevimeline or a derivative thereof.

In some embodiments, the medicament is used for: (a) ameliorating, alleviating, inhibiting and/or relieving pupil photophobia, blurred near vision and/or accommodative eye fatigue in adolescents and adults, and/or (b) ameliorating and improving distance vision in adolescents and adults.

In another aspect, the present disclosure further provides a medicament for treating or preventing myopia, comprising the above-mentioned ophthalmic preparation.

In some embodiments, the medicament is used for: (a) ameliorating, alleviating, inhibiting and/or relieving pupil photophobia, blurred near vision and/or accommodative eye fatigue in adolescents and adults, and/or (b) ameliorating and improving distance vision in adolescents and adults. The active ingredients of the ophthalmic preparation and the corresponding CAS numbers or scientific names or molecular structural formulas are as follows:

| Active ingredient | | English name | CAS number |
|---|---|---|---|
| | Acetylcholine | Acetylcholine | 51-84-3 |
| | Methacholine | Methacholine (chloride) | 62-51-1 |
| | Carbachol | Carbachol | 51-83-2 |
| | Bethanechol chloride | Bethanechol chloride | 590-63-6 |
| | Pilocarpine | Pilocarpine | 92-13-7 |
| Cholinoceptor agonist | Aceclidine | Aceclidine | 827-61-2 |
| | Cevimeline | Cevimeline | 107233-08-9 |
| | Xanomeline | Xanomeline | 131986-45-3 |
| | Sabcomeline | Sabcomeline | 159912-53-5 |
| | Methacholine bromide | Methacholine bromide (RG) | 333-31-3 |
| | Milameline | Milameline | 139886-32-1 |
| | Arecoline | Arecoline | 63-75-2 |
| | Neostigmine | Neostigmine | 59-99-4 |
| | Mestinon | Mestinon | 101-26-8 |
| | Physostigmine | Physostigmine | 57-47-6 |
| | Edrophonium chloride | Edrophonium chloride | 116-38-1 |
| Anticholinesterase drug | Ambenonium chloride | Ambenonium chloride | 115-79-7 |
| | Demecarium bromide | Demecarium bromide | 56-94-0 |
| | Galantamine | Galantamine | 357-70-0 |
| | Tacrine | Tacrine | 321-64-2 |
| | Huperzine A | (-)-Huperzine A | 102518-79-6 |
| | Norepinephrine | Norepinephrine | 51-41-2 |
| | Metaraminol | Metaraminol | 54-49-9 |
| | Phenylephrine | (R)-(-)-Phenylephrine | 59-42-7 |
| | Methoxamine | Methoxamine | 390-28-3 |
| | Oxymetazoline | Oxymetazoline | 1491-59-4 |
| | Naphazoline | Naphazoline | 835-31-4 |
| | Xylometazoline | Xylometazoline | 526-36-3 |
| | Apraclonidine | Apraclonidine | 66711-21-5 |
| | Dexmedetomidine | Dexmedetomidine | 113775-47-6 |
| | Brimonidine | Brimonidine | 59803-98-4 |
| α-adrenoceptor agonist | Dopamine | Dopamine | 51-61-6 |
| | Ephedrine | L-(-)-Ephedrine | 299-42-3 |
| | Pseudoephedrine | Pseudoephedrine | 90-82-4 |
| | Mephentermine | Mephentermine | 100-92-5 |
| | Guanfacine | Guanfacine | 29110-47-2 |
| | Iopidine | Iopidine | 73218-79-8 |
| | Tetrahydrozoline | Tetrahydrozoline | 84-22-0 |
| | Xylazine | Xylazine | 7361-61-7 |
| | Tizanidine | Tizanidine | 51322-75-9 |
| | Moxonidine | Moxonidine | 75438-57-2 |
| | Rilmenidine | Rilmenidine | 54187-04-1 |
| | Atropine | Atropine | 51-55-8 |
| | Atropine methyl bromide | Atropine methyl bromide | 2870-71-5 |
| | Ipratropium bromide | Ipratropium bromide | 22254-24-6 |
| | Anisodamine | Anisodamine | 55869-99-3 |
| | Pirenzepine | Pirenzepine | 28797-61-7 |
| Cholinoceptor antagonist | Mecamylamine | Methyl(2,3,3-trimethy-ltrinorbornan-2-yl)amine | 60-40-2 |
| | Pancuronium bromide | Pancuronium bromide | 15500-66-0 |
| | Propantheline bromide | Propantheline bromide | 50-34-0 |
| | Benactyzine | Benactyzine | 302-40-9 |

### Example 1. Preparation of single-component ophthalmic preparation

An effective dose of a pharmaceutical component was dissolved in a minimal volume of Water for Injection, stirred and dissolved to form a for subsequent use pharmaceutical component solution. Specifically, an appropriate amount of Water for Injection was added into a solution preparation tank, followed by the addition of an amount of an osmotic pressure regulator. The mixture was stirred until completely dissolved. Subsequently, the pharmaceutical component solution was added to the solution preparation tank and homogenized. A sample was then withdrawn to measure the pH. Based on the pH value of the drug solution was detected, a pH regulator was incrementally added to adjust the pH to an in-process control range of the drug solution to 6.0-7.0. Finally, the batch was made up to the final prescription volume with Water for Injection, thoroughly stirred, and filtered to obtain the single-component ophthalmic preparation.

Eye drops containing different concentrations of cevimeline were prepared in Examples 1-1 and 1-2 according to the preparation method of Example 1. The specific parameters of the eye drops are as follows:

| Example | Concentration of cevimeline | pH (regulator) | Osmotic pressure (regulator) |
|---|---|---|---|
| 1-1 | 4 wt% | 6.7 (0.5 mol/L NaOH) | 287 mOsmol/kg (NaCl) |
| 1-2 | 2.25 wt% | 6.7 (0.5 mol/L NaOH) | 281 mOsmol/kg (NaCl) |

### Example 2. Preparation of multi-component ophthalmic preparation

### Preparation of ophthalmic preparation formula 3 (a compound eye drop containing 0.05% of atropine and 2.25% of cevimeline)

An appropriate amount of a pharmaceutical component A and a small amount of Water for Injection were mixed and stirred until dissolved to obtain a solution A for subsequent use, and an appropriate amount of a pharmaceutical component B and a small amount of Water for Injection were mixed and stirred until dissolved to obtain a solution B for later use. An appropriate amount of Water for Injection was added to a solution preparation tank, followed by the addition of a required amount of sodium chloride and stirring until completely dissolved. Subsequently, solution A and solution B were transferred to the tank and homogenized. A sample was withdrawn to measure the pH. Based on the pH, a pH regulator was incrementally added to adjust the pH to an in-process control range of 5.0-7.0. Finally, the batch was made up to the final prescription volume with WFI, thoroughly stirred, and filtered to obtain the A+B dual-component ophthalmic preparation.

Eye drops containing different pharmaceutical components with different concentrations were prepared in Examples 2-1, 2-2 and 2-3 respectively by using the preparation method of Example 2. The specific parameters of the eye drops are as follows:

| Example | Component A | Component B | pH (regulator) | Osmotic pressure (regulator) |
|---|---|---|---|---|
| 2-1 | 0.05 wt% of atropine | 2.25 wt% of cevimeline | 5.5 (0.5 mol/L NaOH) | 293 mOsmol/kg (NaCl) |
| 2-2 | 0.15 wt% of brimonidine tartrate | 2.25 wt% of cevimeline | 6.6 (0.5 mol/L NaOH) | 291 mOsmol/kg (NaCl) |
| 2-3 | 0.0001 wt% of neostigmine | 2.25 wt% of cevimeline | 6.5 (0.5 mol/L NaOH) | 288 mOsmol/kg (NaCl) |

### Comparative example 1

In this Comparative example, a plurality of atropine single-component ophthalmic preparations with different concentrations were prepared by using the preparation method provided in Example 1. The ophthalmic preparations have the following specific compositions:

| Comparative example | Atropine | pH (regulator) | Osmotic pressure (regulator) |
|---|---|---|---|
| 1-1 | 0.01 wt% | 5.6 (0.5 mol/L NaOH) | 288 mOsmol/kg (NaCl) |
| 1-2 | 0.03 wt% | 5.3 (0.5 mol/L NaOH) | 290 mOsmol/kg (NaCl) |
| 1-3 | 0.05 wt% | 5.5 (0.5 mol/L NaOH) | 293 mOsmol/kg (NaCl) |
| 1-4 | 0.1 wt% | 5.5 (0.5 mol/L NaOH) | 290 mOsmol/kg (NaCl) |
| 1-5 | 1 wt% | 5.5 (0.5 mol/L NaOH) | 290 mOsmol/kg (NaCl) |

### Comparative example 2

In this Comparative example 2, a plurality of different ophthalmic preparations were prepared by using the preparation method provided in Example 2. The ophthalmic preparations possess the following specific compositions:

| Comparative example | Component A | Component B | pH (regulator) | Osmotic pressure (regulator) |
|---|---|---|---|---|
| 2-1 | 0.05 wt% of atropine | 0.15 wt% of brimonidine tartrate | 5.5 (0.5 mol/L NaOH) | 290 mOsmol/kg (NaCl) |
| 2-2 | 0.05 wt% of atropine | 0.0001 wt% of neostigmine | 5.5 (0.5 mol/L NaOH) | 292 mOsmol/kg (NaCl) |

### Effect tests

### Test example 1. Investigation of distance vision effects of ophthalmic preparations provided in Examples 1 and 2

Subject information: the subjects were a male subject, about 60 years old, diagnosed with presbyopia and myopia, and a female patient, about 45 years old, diagnosed with myopia. All subjects were in good general health and able to comply with the protocol requirements. They were capable of undergoing all examinations and reporting subjective symptomatic changes. After learning about the trial objectives, the properties of the investigational ophthalmic preparation, potential risks of the trial and their own rights, the subjects voluntarily participated in this trial.

Assessment of Distance Visual Acuity: Distance vision of the patients was measured using a national standard logarithmic eye chart at a distance of 5 m was used as a standard. Under the condition of sufficient light, the best vision performance was 1.0. During testing, the examiner uses a visual acuity pointer to point at the optotypes on the visual acuity chart. The subject is required to strictly occlude either the left eye or the right eye, then state or gesture the gap orientation of the optotypes. Testing is performed line by line to find the best recognizable line of the subject, so as to measure the specific visual acuity of the left eye and the right eye respectively.

One drop of each of the ophthalmic preparations provided in Examples 1 and 2 was dropped into the lower conjunctiva fornix of the subject. The lower eyelid was gently lifted, the eye was closed, the inner canthus was digitally compressed, and the globe was rotated maintained for 1 minute. Distance Visual Acuity was assessed before administration and at predetermined time intervals post-instillation:5 min, 10 min, 15 min, 20 min, 30 min, 1 h, 1.5 h, 2 h, 3 h, 5 h, 7 h, 8 h and 10 h after the administration. The results are shown in the table below:

| Time | Distance vision | | | | |
|---|---|---|---|---|---|
| | Example 1-1 | Example 1-2 | Example 2-1 | Example 2-2 | Example 2-3 |
| | 4 wt% of cevimeline | 2.25 wt% of cevimeline | 0.05 wt% of atropine+2.2 5 wt% of cevimeline | 0.15 wt% of brimonidine tartrate+2.25 wt% of cevimeline | 0.0001 wt% of neostigmine +2.25 wt% of cevimeline |
| 0 min | 0.5 | 0.5 | 0.5 | 0.5 | 0.5 |
| 5 min | 0.6 | 0.5 | 0.4 | 0.6 | 0.6 |
| 10 min | 0.6 | 0.6 | 0.5 | 0.6 | 0.6 |
| 15 min | 0.8 | 0.6 | 0.6 | 0.8 | 0.6 |
| 20 min | 0.8 | 0.8 | 0.6 | 0.8 | 0.8 |
| 30 min | 0.8 | 0.8 | 0.8 | 0.8 | 0.8 |
| 1 h | 0.8 | 0.8 | 0.8 | 0.8 | 0.8 |
| 1.5 h | 0.8 | 0.8 | 0.8 | 0.8 | 0.8 |
| 2 h | 0.8 | 0.8 | 0.8 | 0.8 | 0.8 |
| 3 h | 0.8 | 0.8 | 0.8 | 0.8 | 0.8 |
| 5 h | 0.8 | 0.8 | 0.8 | 0.8 | 0.8 |
| 7 h | 0.8 | 0.8 | 0.8 | 0.8 | 0.8 |
| 8 h | 0.8 | 0.8 | 0.8 | 0.8 | 0.8 |
| 10 h | 0.6 | 0.6 | 0.8 | 0.8 | 0.6 |

Result analysis: As evidenced by the above table that all the ophthalmic preparations provided in Examples 1 and 2 can improve distance vision. The ophthalmic preparation 1-1 and the ophthalmic preparation 2-2 can improve the distance vision by 0.3 at 15 min, the ophthalmic preparation 1-2 and the ophthalmic preparation 2-3 achieved a comparable gain of 0.3 within 20 minutes, the ophthalmic preparation 2-1 exhibited a slower onset, improved the distance vision slowly and the distance vision can be 0.3 after 30 min. Then the therapeutic effects of the ophthalmic preparations 2-1 and 2-2 were sustained throughout the entire 10-hour observation period. These results demonstrate that cevimeline-based ophthalmic preparations provide a rapid onset of action coupled with a durable duration of efficacy in enhancing distance vision.Compared with cevimeline eye drops at identical concentrations, the atropine-cevimeline combination eye drops exhibit slightly slower onset yet markedly prolonged duration.The brimonidine tartrate and cevimeline combination eye drops exhibit a rapid onset of action coupled with a prolonged duration.

### Test example 2. Effect of ophthalmic preparations provided in Comparative example 1 in improving distance vision

Subject information: The study cohort comprised a 14-year-old male subject diagnosed with myopia and a 45-year-old female subject diagnosed with myopia.

All subjects were in good general health and able to comply with the protocol requirements. They were capable of undergoing all examinations and reporting subjective symptomatic changes. After learning about the trial objectives, the properties of the investigational ophthalmic preparation, potential risks of the trial and their own rights, the subjects voluntarily participated in this trial.

One drop of each of the ophthalmic preparations prepared in Comparative example 1 was dropped into the lower conjunctiva fornix of each subject. The lower eyelid was gently lifted, the eye closed, the inner canthus digitally compressed, and the globe rotated; this maneuver was held for 1 minute. The Distance Visual Acuity was assessed before administration and at predetermined time intervals post-instillation: 5 min, 10 min, 15 min, 20 min, 30 min and 1 h after the administration. The results are shown in the table below:

| Embodiments | Distance vision after 1 h | Blurred vision or not | Photophobia or not |
|---|---|---|---|
| Comparative example 1-1 | Not improved | Yes | No |
| Comparative example 1-2 | Not improved | Yes | No |
| Comparative example 1-3 | Not improved | Yes | Yes |
| Comparative example 1-4 | Not improved | Yes | Yes |
| Comparative example 1-5 | Not improved | Yes | Yes |
| Example 2-1 | Improved by 0.3 | No | No |

The results demonstrated that, within 1h, subjects receiving the Comparative examples showed blurred vision. They cannot focus on the distance vision chart. Consequently, the distance vision of the subjects was not continuously observed.

### Test example 3. Comparison of the regulation of eye diopters and axis oculi by ophthalmic preparations provided in different embodiments

Establishment of a form-deprivation myopia model: the right eyes of chicken in an experimental group were masked and the left eyes were used as the self-control eyes. Both eyes of a normal control group and the control eyes of the experimental group were not treated. After the chicken were raised for 4 weeks, whether the covers were displaced or the control eyes were covered was periodically checked. The refractive state of the eyes and the length of the axis oculi of the chicken were measured at the end of week 4.

Administration scheme: a total of 5 groups, wherein Group 1 was a model group, Group 2 was a model group + eye drop of Comparative example 1-3, Group 3 was a model group + eye drop of Example 1-2, Group 4 was a model group + eye drop of Example 2-1, and Group 5 was a model group + eye drop of Example 2-2. After the chicken were raised for 4 weeks, binocular refractive state (D) and axis oculi (mm) were detected to evaluate the accommodation of the different preparations on myopia. The results are shown in the table below:

| | Group 1 | Group 2 | Group 3 | Group 4 | Group 5 |
|---|---|---|---|---|---|
| Refractive states of both eyes | -16.4±1.7 | -9.2±3.1^{*} | -14.7±5.2 | -8.9±2.9^{*} | -15.0±4.2 |
| Axis oculi (mm) | 0.43±0.1 9 | 0.19±0.12 ^{*} | 0.40±0.1 6 | 0.20±0.17 ^{*} | 0.41±0.1 5 |

Compared with the model group, * represents p < 0.05. There is no significant difference between Group 2 and Group 4.

As shown in the table above, compared with the model group, both Comparative example 1-3 (0.05 wt% of atropine) and Example 2-1 (2.25 wt% of cevimeline + 0.05 wt% of atropine) can effectively improve the diopter and inhibit axial elongation. However, neither Example 1-2 (2.25 wt% of cevimeline) nor Example 2-2 (2.25 wt% of cevimeline and 0.15 wt% of brimonidine tartrate) demonstrated improvement in diopter or inhibition of axial elongation axis oculi. Furthermore, the results of Comparative Example 2 demonstrated that ocular instillation of atropine induced significant adverse effects, including blurred vision and photophobia. These adverse effects were avoided in the formulations comprising cevimeline. Besides, the use was not constrained by specific schedules or environmental limitations, thereby affording high patient compliance and considerable convenience. Therefore, the combined preparation of the atropine and the cevimeline provided in Example 2-1 was required to be selected for the long-term effective prevention and control of the occurrence and development of juvenile myopia.

The above detailed description is specific to one of the feasible examples of the present disclosure. The examples are not intended to limit the scope of the present disclosure. Any equivalent implementation or modification without departing from the present disclosure should be included in the scope of technical solutions of the present disclosure.

## Claims

1. An ophthalmic preparation for treating and/or ameliorating myopia, wherein the ophthalmic preparation comprises cevimeline.

2. The ophthalmic preparation according to claim 1, wherein the ophthalmic preparation further comprises at least one of another cholinoceptor agonist, an anticholinesterase drug, an α-adrenoceptor agonist, or a cholinoceptor antagonist.

3. The ophthalmic preparation according to claim 2, wherein the cholinoceptor agonist comprises acetylcholine, methacholine, carbachol, bethanechol chloride, pilocarpine, aceclidine, cevimeline, xanomeline, sabcomeline, methacholine bromide, milameline or arecoline, or a derived compound possessing the same function thereof.

4. The ophthalmic preparation according to claim 2 or 3, wherein the anticholinesterase drug comprises neostigmine, mestinon, physostigmine, edrophonium chloride, ambenonium chloride, demecarium bromide, galantamine, tacrine, huperzine A or a derived compound having the same function thereof, and is optionally neostigmine.

5. The ophthalmic preparation according to any one of claims 2-4, wherein the α-adrenoceptor agonist includes norepinephrine, metaraminol, phenylephrine, methoxamine, oxymetazoline, naphazoline, xylometazoline, apraclonidine, dexmedetomidine, brimonidine, dopamine, ephedrine, pseudoephedrine, mephentermine, guanfacine, Iopidine, tetrahydrozoline, xylazine, tizanidine, moxonidine, rilmenidine, or functionally equivalent derivatives thereof, and is optionally brimonidine.

6. The ophthalmic preparation according to any one of claims 2-5, wherein the cholinoceptor antagonist comprises atropine, atropine methyl bromide, ipratropium bromide, anisodamine, pirenzepine, mecamylamine, pancuronium bromide, propantheline bromide, benactyzine, or functionally equivalent derivatives thereof, and is optionally atropine.

7. The ophthalmic preparation according to any one of claims 1-6, wherein the concentration of the cevimeline is 0.1-10 wt%, preferably 0.5-8 wt%, further preferably 1-5 wt%, and further preferably 2-3 wt%.

8. The ophthalmic preparation according to any one of claims 1-7, wherein the anticholinesterase drug is neostigmine and the concentration of the neostigmine is 0.0001-0.5 wt%, preferably 0.0001-0.1 wt%, further preferably 0.0001-0.05 wt%, further preferably 0.0001-0.01 wt%, and further preferably 0.0001-0.001 wt%.

9. The ophthalmic preparation according to any one of claims 1-8, wherein the α-adrenoceptor agonist is brimonidine and the concentration of the brimonidine is 0.05-1.0 wt%, preferably 0.05-0.5 wt%, further preferably 0.05-0.2 wt%, and further preferably 0.1-0.2 wt%.

10. The ophthalmic preparation according to any one of claims 1-9, wherein the cholinoceptor antagonist is atropine and the concentration of the atropine is 0.01-1.0 wt%, preferably 0.01-0.5 wt%, preferably 0.01-0.1 wt%, and preferably 0.03-0.07 wt%.

11. The ophthalmic preparation according to any one of claims 1-10, wherein the ophthalmic preparation is used for ameliorating, alleviating, inhibiting and/or relieving pupil photophobia, blurred near vision and/or accommodative eye fatigue in adolescents and adults.

12. The ophthalmic preparation according to any one of claims 1-10, wherein the ophthalmic preparation is used for ameliorating and improving distance vision in adolescents and adults.

13. The ophthalmic preparation according to any one of claims 1-12, wherein the ophthalmic preparation further comprises a pharmaceutically acceptable carrier and/or excipient; and
preferably, the excipient comprises at least one of a pH regulator, an osmotic pressure regulator or a buffer.

14. The ophthalmic preparation according to any one of claims 1-13, wherein a dosage form of the ophthalmic preparation comprises an eye drop, an eye gel, an eye ointment, an eye patch, an eye film, an eye wash or an intraocular injection.

15. Use of cevimeline or a derivative thereof in the manufacture of a medicament for treating or preventing myopia.

16. The use according to claim 15, comprising: (a) ameliorating, alleviating, inhibiting and/or relieving pupil photophobia, myopia blurring and/or accommodative eye fatigue in adolescents and adults, and/or (b) ameliorating and improving distance vision in adolescents and adults.

17. Use of the ophthalmic preparation according to any one of claims 1-14 in the manufacture of a medicament for treating or preventing myopia.

18. The use according to claim 17, wherein the use comprises: (a) ameliorating, alleviating, inhibiting and/or relieving pupil photophobia, myopia blurring and/or accommodative eye fatigue in adolescents and adults, and/or (b) ameliorating and improving distance vision in adolescents and adults.

19. A method for preparing the ophthalmic preparation according to any one of claims 1-14, wherein the preparation method comprises adding cevimeline or a derivative thereof into a solvent and making up to a volume to obtain the ophthalmic preparation;
preferably, before making up to the volume, further adding at least one of another cholinoceptor agonist, an anticholinesterase drug, an α-adrenoceptor agonist, or a cholinoceptor antagonist; and
preferably, before making up to the volume, further adding a pharmaceutically acceptable carrier and/or excipient.

20. A medicament for treating or preventing myopia, comprising cevimeline or a derivative thereof.

21. The medicament according to claim 20, wherein the medicament is used for: (a) ameliorating, alleviating, inhibiting and/or relieving pupil photophobia, myopia blurring and/or accommodative eye fatigue in adolescents and adults, and/or (b) ameliorating and improving distance vision in adolescents and adults.

22. A medicament for treating or preventing myopia, comprising the ophthalmic preparation according to any one of claims 1-14.

23. The medicament according to claim 22, wherein the medicament is used for: (a) ameliorating, alleviating, inhibiting and/or relieving pupil photophobia, myopia blurring and/or accommodative eye fatigue in adolescents and adults, and/or (b) ameliorating and improving distance vision in adolescents and adults.

24. A method for treating or preventing myopia, comprising administering to a subject a therapeutically effective amount of the ophthalmic preparation according to any one of claims 1-14.
